# EUROPEAN PATENT SPECIFICATION

(11) **EP 0 292 535 B1**
(45) Date of publication and mention of the grant of the patent: **23.06.1993**
(21) Application number: 88900078.2
(22) Date of filing: 04.12.1987
(51) Int. Cl.: G01N 33/543, G01N 33/52

(54) **SOLID PHASE IMMUNOASSAY**
FESTPHASENIMMUNOASSAYVERFAHREN
IMMUNO-ANALYSE EN PHASE SOLIDE

(30) Priority: 04.12.1986 GB 8629001; 29.06.1987 GB 8715226; 29.06.1987 GB 8715227
(43) Date of publication of application: 30.11.1988
(73) Proprietor: KEMENY, David Michael, London SE11 6BY (GB)
(72) Inventor: KEMENY, David Michael, London SE11 6BY (GB)
(74) Representative: Beresford, Keith Denis Lewis
(86) International application number: GB8700878
(87) International publication number: WO8804430

(56) References cited:
- EP-A- 63 810
- EP-A- 132 170
- FR-A- 2 369 557
- FR-A- 2 556 840
- FR-A- 2 599 844

## Description

This invention relates in one aspect to a method of solid phase immunoassay.

Several different types of solid phase immunoassay are currently used. One example is RAST (radio allergosorbent test). Typically, in RAST, cellulose paper discs are used as the carriers, to which an allergen is bound. A sample of the test liquid containing antibodies to the allergen is added to the disc in a microtitre plate and the plate is incubated, for example for eighteen hours. For a disc having a liquid capacity of 3 µl, for example, the sample volume is typically 50 µl. After washing and removal of surplus liquid, 50 µl of a liquid containing a radio labelled indicator specific to the antibody to be assayed is added to the disc in the plate, and a further incubation is carried out for eighteen to twenty four hours. After washing the disc, its residual radioactivity is counted to give a measure of the quantity of the antibody bound to the disc, and thus the quantity in the original sample.

RAST is a sensitive test, particularly useful for allergen-specific antibodies, and is easily adapted to a wide range of allergens. A disadvantage, however, is the relatively long time required to conduct the test. An other solid phase immunoassay the PRIST (paper radio immunosorbent test, in which an antibody to IgE is bound to the paper disc), also requires significant incubation time to achieve satisfactory sensitivity.

It has now been found that by reducing the volume of the liquid in which the indicator is contained, relative to the liquid capacity of the disc, a significant increase in sensitivity can be achieved, permitting positive detection of a lower concentration of analyte in a sample than with conventional tests, and the incubation time with the indicator liquid can also be reduced significantly.

It has also been found that if the volume of sample liquid is also reduced, relative to the liquid capacity of the disc, the incubation time can be reduced to as little as five minutes while achieving a sensitivity comparable with that of conventional tests.

In GB-A-1420916, there is disclosed a radio-immunoassay test conducted in glass fibre filter material using 0.1 ml of liquid, which is approximately equal to the filter material capacity. However, the test is of the competitive type, using a significantly larger volume of liquid than the tests of the type with which the present invention is concerned.

EP-A-0132170 discloses a method of immunoassay in which there is produced an antibody/antigen/antibody complex with the first antibody being attached to a solid support and in which volumes in the range 10 to 200 µl and incubation periods of some minutes to some hours are stated to be used. However, there is no disclosure that reduction of indicator volume can lead to increase in sensitivity, and the incubation times exemplified are in hours rather than minutes. EP-A-0063810 and FR-A-2369557 also disclose methods for immunoassay based on solid supports.

The invention provides a method of carrying out a radio allergosorbent test (RAST test), or a paper radio immunoabsorbent test in which an antibody to IgE is bound to a paper disc (PRIST test), comprising contacting a sample liquid containing an analyte with an absorbent carrier having a binding material immobilised therein or thereon, whereby analyte in the liquid becomes bound to the binding material, allowing a radioactivity labelled indicator to contact the bound analyte, whereby indicator becomes bound to the bound analyte, removing unbound indicator from the carrier, and detecting the remaining bound indicator by detecting the radioactivity thereof, the labelled indicator being contained in a volume of liquid less than ten times the liquid saturation volume of the carrier, characterised in that:
(a) the volume of indicator is not more than 25 µl; and
(b) the concentration of labelled indicator used is such that the amount of indicator present in the volume of liquid is the same as the amount used in a RAST or PRIST test.

The volume of indicator liquid is preferably less than five times the saturation volume of the carrier, more preferably less than twice the saturation volume. Most preferably, the volume of liquid is substantially equal to the saturation volume of the carrier which is suitably a cellulose paper carrier.

Where the concentration of the analyte is very low, but quantities are not restricted, for example in tissue culture supernatants, umbilical cord blood samples, and body fluids such as saliva and nasal washings, the volume of sample liquid added and the incubation time are suitably chosen to be comparable with those in conventional RAST or PRIST tests.

Where a higher concentration of analyte is present in the sample liquid, for example antibodies in serum, a volume of sample liquid less than ten times the saturation volume of the carrier is used. Preferably less than five times the saturation volume is used, more preferably less than twice the saturation volume. Most preferably, a volume substantially equal to the saturation volume is used. With reduced volumes of the sample liquid, substantially smaller incubation times are required, and the assay may therefore be completed in a substantially shorter time than is required for conventional tests, for example as little as 65 minutes for IgE antibodies.

The use of small sample volumes in accordance with the invention permits economical use of reference reagents. This makes it possible to hold supplies of a wide range of reference reagents, in particular serum samples, which is impractical for conventional RAST assay methods using large volumes of sample.

Accordingly, the invention also provides an immunoassay kit for use in the immunoassay method of the invention, comprising a plurality of absorbent carrier members having a binding material immobilised therein or thereon, a supply of a solution containing a labelled indicator, and a plurality of different reference samples containing known concentrations of known analytes.

In non-competitive tests such as RAST it is advantageous or essential to wash the carrier discs after each incubation period, and a known washing device for this purpose has a plate which can be mounted between a washing liquid supply and a vacuum chamber, the plate having a plurality of wells, each of which receives a filter disc, the device permitting a plurality of discs to be washed simultaneously. The discs remain in the wells during each stage of the processing, but are removed for the radioactive testing step. Typically, 96 wells are provided in each plate, permitting 96 discs to be processed simultaneously in each batch. While the conventional washing device permits rapid processing of the batches, the testing step requires the careful removal of each disc from its well, and this can slow the operation.

WO82/03690 discloses apparatus for carrying out e.g. immunoassay reactions in which porous bottomed reaction vessels provided with a filter for retaining insoluble particles fit into wells within a holder and in which suction lines in connection with the wells enable fluid to be supplied to or removed from the reaction vessels.

Accordingly, a preferred washing device when used in the method of this invention is characterised in that the holding plate is removably mountable between the supply and the receiving chamber.

In one form of the washing device, a holder is removably located in each well, the holder having a drain hole therein corresponding with the outlet, and the washing liquid supply is arranged to supply liquid to the holders.

Preferably, the drain hole in each holder is sized such that liquid in the holder is not lost therethrough under gravity. The holders may be in the form of cups or tubes with a drain hole in the base thereof. The cups or tubes are suitably formed of plastics material or glass and are dimensioned and arranged to be a close, but readily removable, fit within the wells. The outlets from the wells are suitably capillaries of the same size as the drain holes in the holders.

The holding plates are more readily handled than the individual filter discs they contain, and thus the processing of batches of filters is facilitated. In some circumstances the use of holders in the wells is preferred. Each holder may, if desired, be suitably labelled to identify an individual disc.

Reference is made to the drawings, in which:
Figure 1 is a series of graphs illustrating the effect of reducing sample volumes (Figure 1A) indicator volume (Figure 1B) and sample and indicator volumes (Figure 1C), and increasing indicator concentration with reduced volumes of indicator and sample (Figure 1D);
Figure 2 is a graph showing the correlation between the results obtainable by the method of the invention and those obtainable by conventional RAST;
Figure 3 is a pair of graphs showing rate of binding of analyte (IgE antibody) (Figure 3A) and a rate of binding of indicator (¹²⁵I anti-IgE) (Figure 3B) for conventional RAST;
Figure 4 is a pair of graphs showing the corresponding rates for the assay of the invention;
Figure 5 is a graph illustrating the effect of sample volume on the sensitivity of an assay in accordance with the invention;
Figure 6 is a pair of graphs illustrating the effect of reduced indicator volume on sensitivity at three sample volumes (Figure 6A), compared with conventional RAST procedure (Figure 6B).
Figure 7 is a series of graphs showing the rate of binding of IgG antibody with various dilutions of high titre (Figure 7A) and low titre (Figure 7B) IgG antibody samples, and the rate of binding of the indicator (¹²⁵ I anti IgG) for the same dilutions of high and low titre samples (Figure 7C and 7D);
Figure 8 is a pair of graphs showing the effect of sample volume (Figure 8A) and ¹²⁵I anti IgG volume (Figure 8B) in assay of IgG antibody to bee venom according to the invention;
Figure 9 is a graph showing the effect of reducing ¹²⁵I anti-IgE volume in PRIST;
Figure 10 shows a washing device for use with the method of the invention, with the holding plate removed therefrom; and
Figures 11 and 12 are respectively side elevation and top plan views of the holding plate for the device shown in Figure 11.

For the following examples, serum samples were collected from patients allergic to bee venom, grass pollen and castor bean, which had previously be shown to contain IgE antibodies. For most experiments a pool of positive sera was used.

### Preparation of ¹²⁵I anti-IgE

Rabbit anti-IgE was prepared by immunisation of New Zealand white rabbits with 0.25 mg IgE PS (from myeloma patient PS) incomplete Freund's adjuvant intra-muscularly followed by three two-weekly boosts with 0. 25 mg IgE PS in incomplete Freund's adjuvant.

The serum was separated and absorbed extensively with normal human serum (20 ml serum/10 ml CNBr-Sepharose 4B® and human gamma globulin (100 mg/10 ml sepharose 4B® (agarose). The anti IgE was then affinity purified over a column containing IgE WT (from myeloma patient WT). Agarose (10 mg/10ml CNBr-sepharose 4B®). the purified antibody was labelled with ¹²⁵ NaI, supplied by Amersham International PLC, UK.

### COMPARATIVE EXAMPLE 1

### RAST

In a conventional RAST method, allergen coated discs having a liquid capacity of 3 µl where washed with phosphate buffered saline (PBS) 0.05 M, pH 7.4 + 0.05% Tween® 20 in a known washing device as hereinbefore described, which removes all surplus liquid from the washed discs. The test sample (50 µl), diluted, if necessary, in horse serum, was incubated with an allergen coated disc in a flat bottomed microtitre plate, (Linbro, Flow Labs Limited, UK) for eighteen hours. The discs were washed as before and 10 ng ¹²⁵I anti-IgE (50 µl), prepared as hereinbefore described, were added. The following day the discs were again washed and the residual radio acitivity counted for one minute in a multi channel gamma counter (LKB-Sweden). Intra-assay variation at 1/5 dilution of serum pool equals 5.7%, n=14.

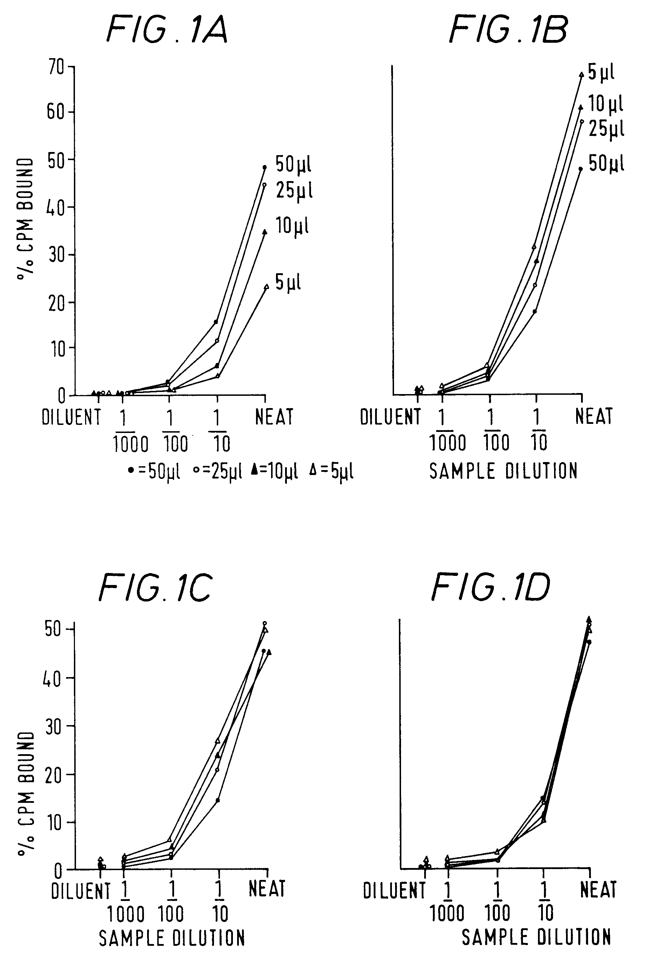

The test was repeated using 25 µl, 10 µl and 5 µl of the test sample, with all the other conditions remaining the same. Further tests were then conducted using test samples diluted to a tenth, a hundredth and a thousandth of the original strength. The results are plotted in Figure 1A. It will be seen that the reduction in sample volume produces a reduction in the number of counts measured for each dilution, ie. sensitivity of the test is reduced when sample volume is reduced.

### Example 1

The tests conducted in the Comparative Example were repeated, keeping the sample volume constant at 50 µl and varying the indicator volume from 50 µl down to 5 µl in the same steps for varying dilutions of sample. The concentration of ¹²⁵I anti-IgE was increased ten fold so that 10 ng was added in 5 µl. It may be seen from Figure 1B that reducing the ¹²⁵ I anti-IgE volumes increased the sensitivity of the test, ie. increased the number of counts per minute measured.

### Example 2

Using the same first and second incubation volumes (5 + 5, 10 + 10, 25 + 25 and 50 + 50) it was possible to achieve comparable results with as little as 5 µl of sample (Figure 1C). The use of small volumes of ¹²⁵I anti-IgE at the same concentration as it is normally used in RAST (0.4 µg/ml) resulted in low radio active counts bound, which necessitated prolonged counting.

### Example 3

The tests conducted in Example 2 were repeated, raising the concentration of the ¹²⁵ I anti-IgE to 4 µg/ml. Similar results were obtained, and the 5 µl assay compares very favourably with the conventional assay procedure using 50 µl of sample and of indicator (Figure 1D).

### COMPARATIVE EXAMPLE 2

Forty eight different samples of sera taken from patients allergic to castor bean were tested by the conventional RAST.

### Example 4

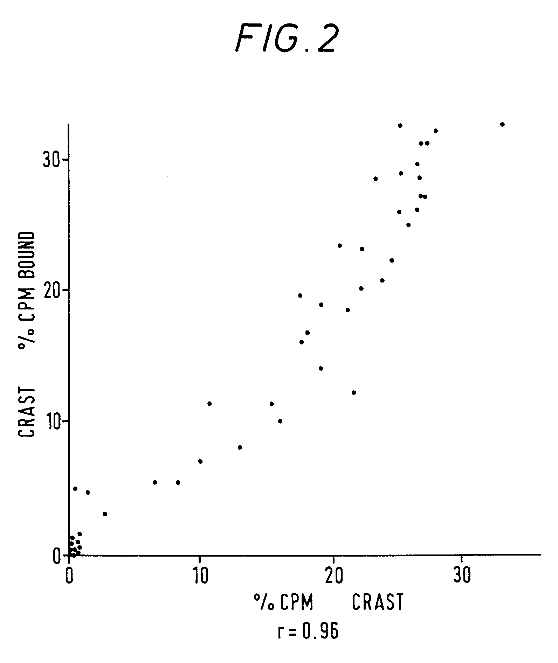

The tests conducted in Comparative Example 2 were repeated using the method of the present invention, as described in Example 3, and for each sample, the counts per minute measured in the conventional test were plotted against the counts per minute measured in the test according to the invention (Figure 2). It will be seen from Figure 2 that a close correlation is obtained between the two methods (r = 0.96).

### COMPARATIVE EXAMPLE 3

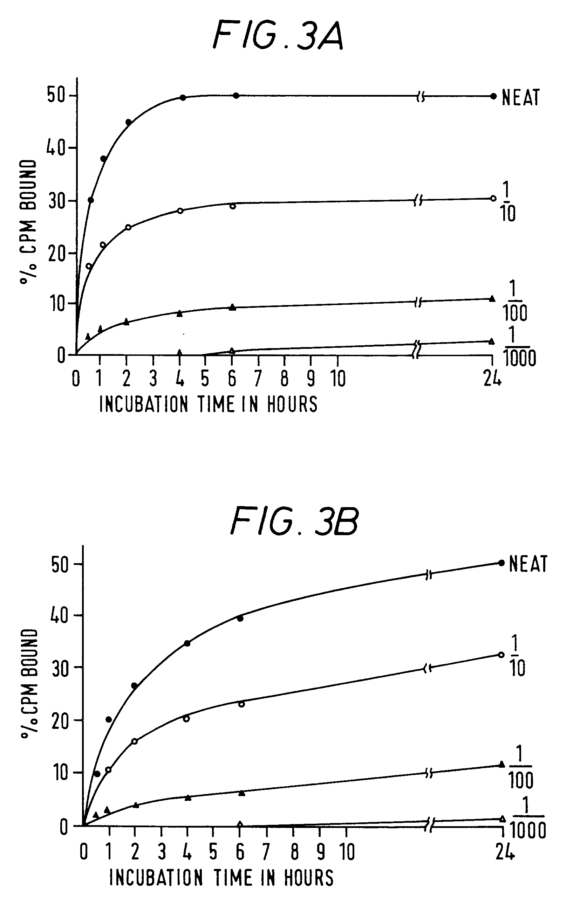

Tests were conducted using conventional RAST to determine the increase in rate of binding of IgE antibody with time at different dilutions of the sample. The results are plotted in Figure 3A. It can be seen that in the first incubation, the time taken for IgE antibody to bind is greater at high than at low dilution, but that even at low dilution it may take four to five hours for equilbrium to be reached.

### COMPARATIVE EXAMPLE 4

Tests were also conducted in accordance with conventional RAST, measuring the rate of binding of ¹²⁵ I anti-IgE at varying dilutions of the indicator, the results being plotted as Figure 3B. The rate at which the ¹²⁵ I anti-IgE binds is normally very slow and a plateau is not reached even after twenty-four hours.

### Example 5

The tests of Comparative Example 3 were repeated using the method of the present invention, varying the dilution of the IgE antibody serum sample. The results are plotted as Figure 4A, and it may be seen that equilbrium is reached very quickly at each dilution. In the method of the present invention, the distances which the antibodies have to diffuse before coming into contact with the antigen are much shorter, and this is reflected in the increased rate at which the IgE antibody binds, as compared with the conventional assay (Figure 3A). Equilibrium is reached within ten minutes, and subsequent experiments have shown that the plateau is reached in about five minutes. With a smaller sample, say 3 µl, it is expected that equilibrium would be reached in less than five minutes.

### Example 6

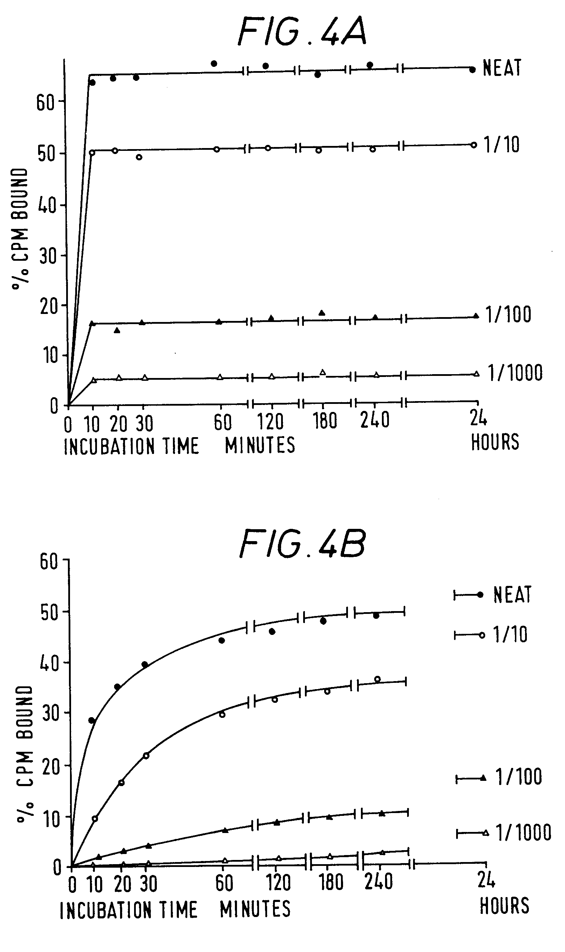

The test conducted in Comparative Example 4 was repeated using the method of the present invention. The rate of binding of the ¹²⁵ I anti-IgE is much more rapid, as may be seen from Figure 4B, than in the conventional test (Figure 3B) both with 0.4 and 4 µg/ml of ¹²⁵ I anti-IgE.

### Example 7

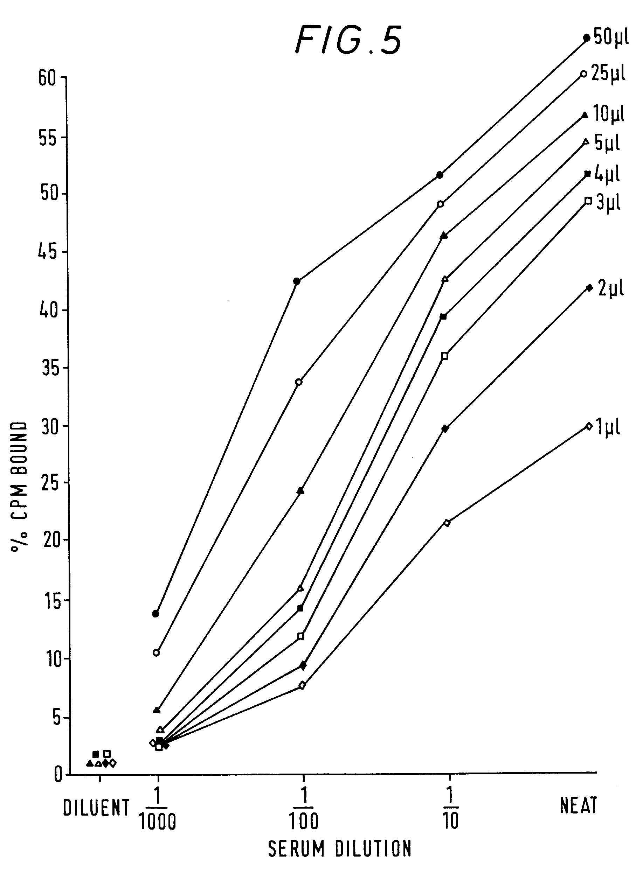

To determine the absolute lower limit to the sample size in the method of the present invention, a wider range of volumes from 50ml down to 1 µl were used with a fixed volume of ¹²⁵ I anti-IgE (5 µl). The results are plotted in Figure 5. For practical purposes, the lower limit can be seen to be about 3 µl. At greater than 5 µl, sensitivity was increased significantly, especially at high sample dilution.

### Example 8

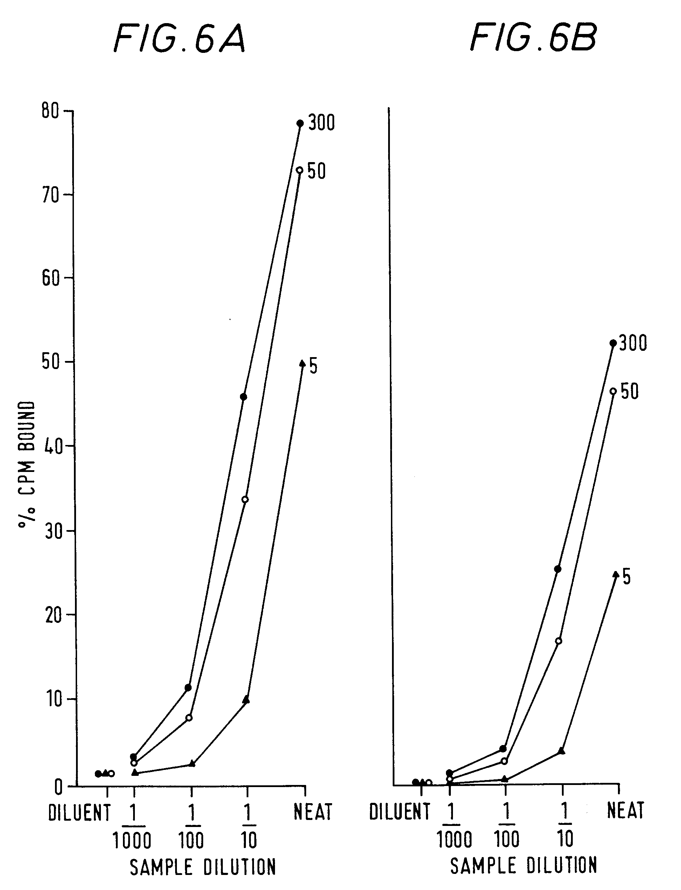

Tests using reduced ¹²⁵ I anti IgE indicator volume (5 µl) in accordance with the invention and using different serum sample volumes at different dilutions were conducted. The results were plotted as Figure 6A, and it can be seen that, using 300 µl of neat sample, up to 80% of added radioactive indicator could be bound.

### COMPARATIVE EXAMPLE 5

The tests of Example 8 were repeated using 50 µl of the indicator with the same quantity of antibody and therefore the same number of radioactive counts as in the 5 µl quantity of indicator used in Example 8. It can be seen from Figure 6B that the sensitivity is substantially reduced, with 300 µl of sample giving only slightly higher number of counts than that for 5 µl in the test according to the invention (Figure 6A).

The increased sensitivity that results from bringing ¹²⁵I anti-IgE into closer contact with the IgE antibody bound to the disc, and the greater efficiency with which IgE antibody can be bound, makes it possible to use very small amounts of serum (as little 3 µl) with equal sensitivity to that obtained in conventional tests. The method of the invention has been used to assay total IgE and IgE antibodies in blood samples taken from babies by heel prick. The same economy of serum is also useful when testing rats, where serum is also in short supply.

### Example 9

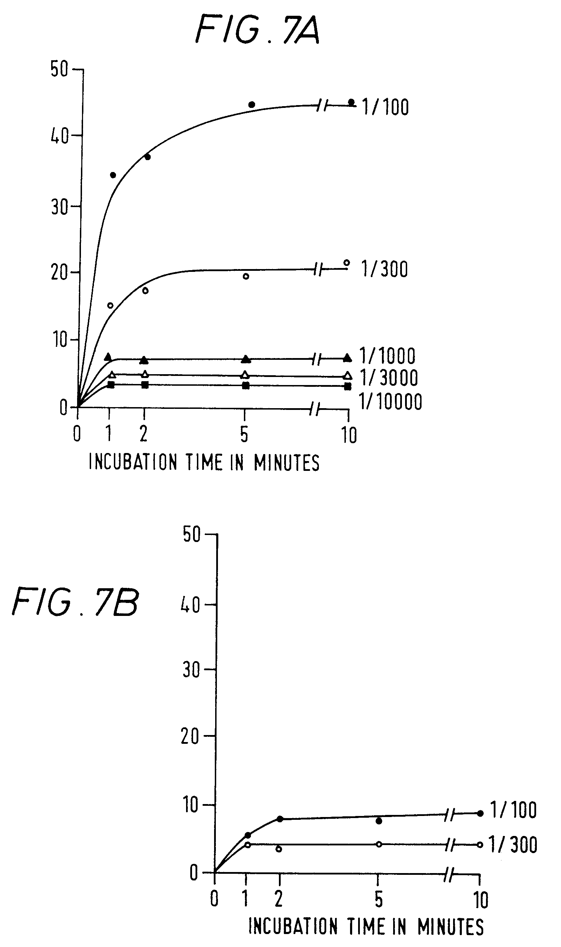

A series of measurements of IgG antibodies to bee venom in serum samples of high and low titre was carried out using cellulose paper discs of about 3 µl liquid capacity coated with bee venom. The discs were incubated with samples at dilutions from 1/100 to 1/10000 for the high titre samples and of 1/100 and 1/300 for the low titre samples. A range of incubation times was used, and at the end of the incubation each sample was washed and then incubated with ¹²⁵I anti-IgG (Pharmacia (GB) plc), followed by further washing. The residual radioactivity was counted in a gamma counter, the results being plotted as Figure 7A (high titre) and Figure 7B (low titre). It can be seen that for high dilutions, a plateau is reached very quickly, within about one minute, while at lower dilutions for the high titre sample the time taken is about five minutes.

### Example 10

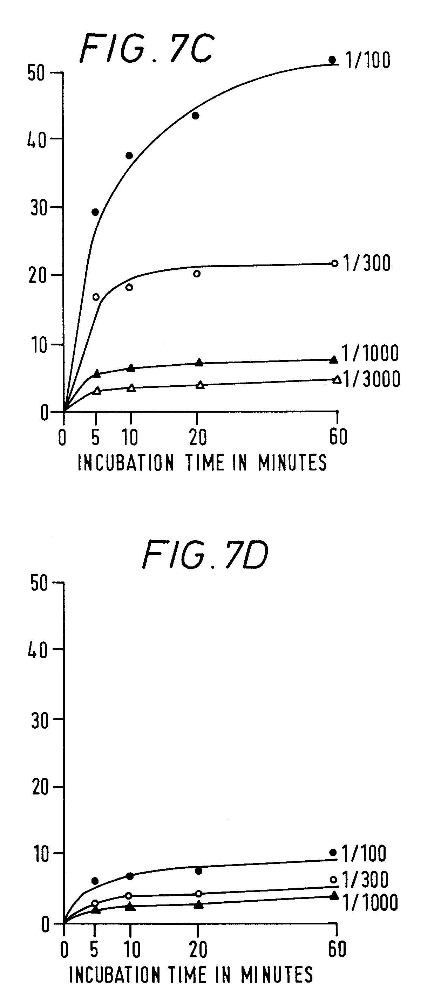

A further series of measurements of IgG antibodies to bee venom in serum samples of high and low titre was carried out as in Example 9, but with a constant incubation time, sufficient to achieve equilibrium. The ¹²⁵ I anti-IgG indicator was used as in Example 9 and the counts measured for various different incubation times. The results are plotted in Figure 7C (high titre) and Figure 7D (low titre). It will again be seen that equilibrium is reached rapidly, especially for higher dilutions of sample, being within about ten minutes.

### COMPARATIVE EXAMPLE 6

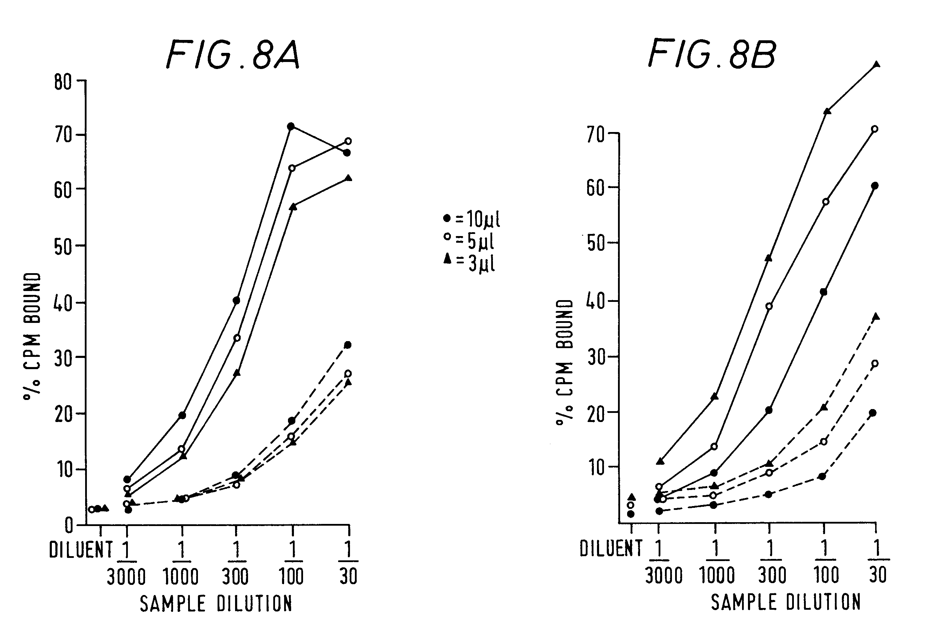

A procedure similar to that of Example 9, but using different quantities of serum, each of several different dilutions and a constant incubation time sufficient to ensure equilibrium, was used to produce a series of graphs for high (solid lines) and low (broken lines) titres of serum. The graphs are shown as Figure 8A, and it can be seen that, with lower quantities, sensitivity is reduced.

### Example 11

By contrast, a series of tests similar to those of Comparative Example 6 were carried out, but using a constant quantity of serum, in varying dilutions, and constant incubation times, while using different volumes of ¹²⁵I I anti-IgG.

It can be seen from Figure 8B that, where the ¹²⁵ I anti-IgG volume is reduced to 3 µl, there is a further increase in sensitivity so that up to 83% of added counts bind.

### Example 12

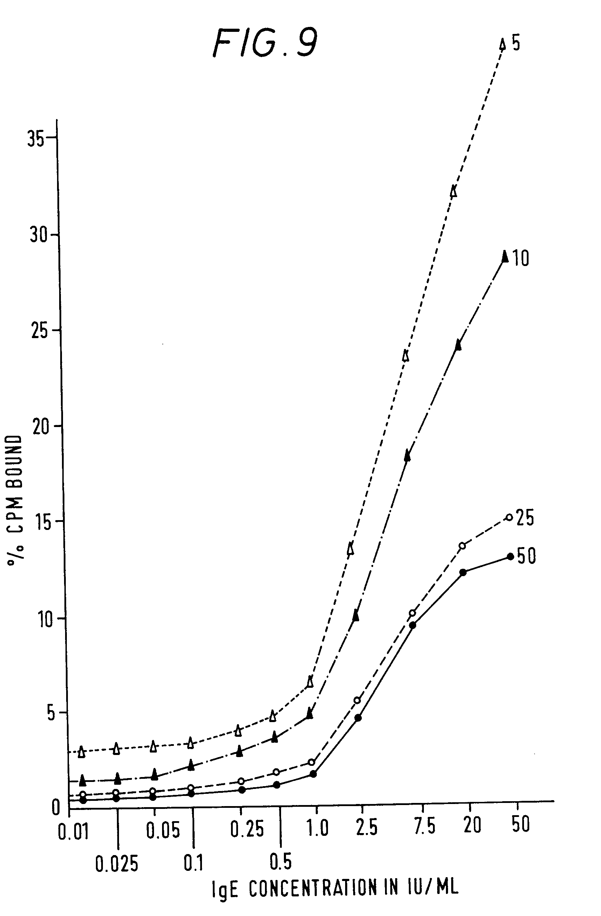

A PRIST assay was carried out in accordance with the invention. PRIST is a 2-site assay in which a cellulose paper disc is coated in anti-IgE which binds all the IgE in the sample. Specific bound IgE is then detected using a radiolabelled anti-IgE indicator. (An enzyme-labelled anti-IgE can be used as an alternative). Sample and indicator volumes of 5 µl, 10 µl and 25 µl were used with a disc of 3 µl capacity. For comparison, a test with 50 µl was also conducted. A range of different IgE concentrations in the sample was tested. The results are shown in Figure 9. It will be seen that, in terms of signal-to-noise, the results obtained using 5 µl are as good as those obtained with 50 ul, but with a much higher signal level in the case of the 5 µl sample.

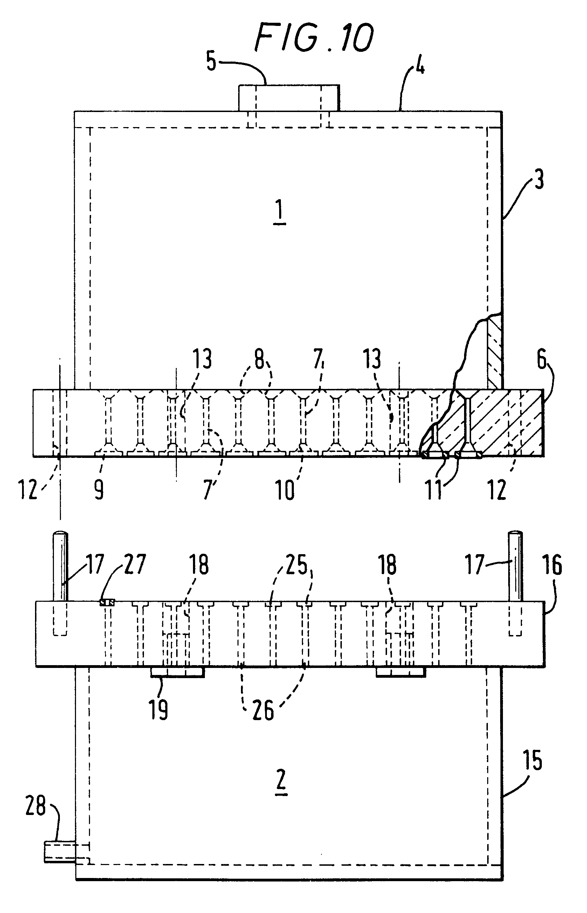

Referring now to the washing device illustrated by Figures 10 to 12, and in particular first to Figure 10, the washing liquid supply chamber 1 is in the form of a rectangular perspex tank 3 having a sealed lid 4 through which there is an opening 5 for introducing the washing liquid. The chamber 1 has a thickened base plate 6 through which passageways 7 extend to provide washing liquid outlets. The passageways 7 are arranged in the base plate in an evenly spaced 8 by 12 array, similar to the arrangement of wells in a conventional microtitre plate. The internal opening of the passageways 7 are formed by conical recesses 8 machined into the upper surface of the base plate 6, and the external ends of the passageways 7 communicate with shallow cylindrical recesses 9 in a lower surfaces of the base plate 6. The shallow cylindrical recesses 9 provide seatings for synthetic rubber o-rings 11 which act as seals.

The end overlaps of the base plate 6 have bore holes 12 extending through them, two at one end and one at the other, to receive locating pegs, while the edge overlaps have bore holes 13 to receive clamping bolts to enable the supply chamber to be clamped to the receiving chamber 2.

The receiving chamber 2 is of similar construction to the supply chamber 1, essentially consisting of a closed rectangular perspex washing liquid receiving tank 15 having a thickened top plate 16 with an 8 by 12 array of drain passages 26 corresponding to the passageways 7. The upper end of each drain passage 26 opens into a recessed 25 in which a rubber o-ring 27 is seated to act as a seal. The end overlaps of the top plate 16 are provided with up-standing pegs 17, two at one end and one at the other, which locate in the bore holes 12 when the chambers 1 and 2 are clamped together with the holding plate therebetween. The edges of the overlaps of the top plate 16 have bore holes 18 extending through them, their lower ends being fitted with bushes 19 to receive the clamping bolts.

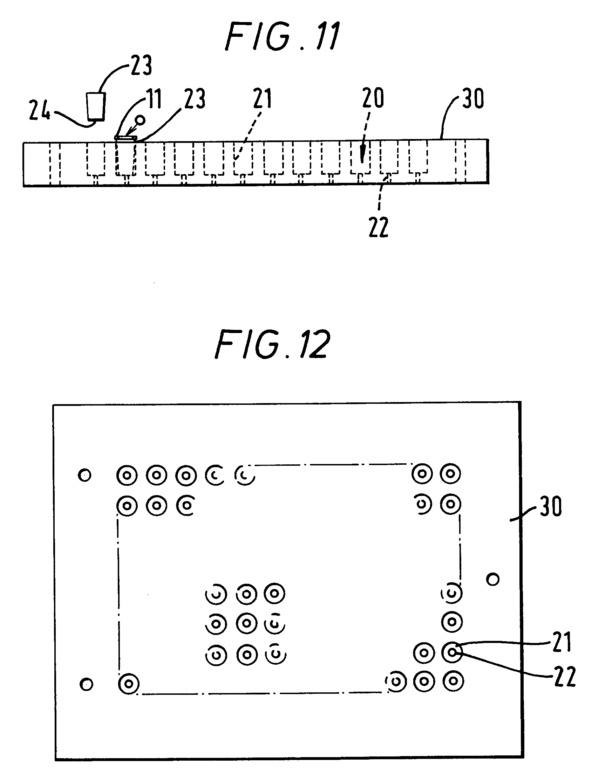

Referring to Figures 11 and 12, the holding plate 30 comprises a rectangular perspex block having an evenly spaced 8 by 12 array of wells 20 corresponding in position to the passageways 7 in the base plate 6 of the supply chamber 1. Each well 20 comprises a cylindrical bore 21, extending through a major part of the thickness of the plate 30, and a capillary 22 extending through the remainder of the thickness of the plate 30. In each well 20 a cup 23, having a capillary hole 24 in the base thereof, may be removably seated. The holding plate 30 has bore holes corresponding with those of the base plate 6 to ensure accurate location of the plate 30 between the chambers 1 and 2.

The interior of the washing liquid receiving chamber 2 is provided with an outlet 28 towards it base, constituting an outlet for spent washing liquid and for applying suction to the chamber. The outlet 28 is conveniently attached direct to a vacuum line connected to vacuum source, preferably by way of a trap to receive spent washing liquid.

When the supply chamber and the receiving chamber are clamped together with the holding plate therebetween, the o-rings 11 are brought to bear on the upper face of the plate 30 or on the rims of the cups 23, if used, thereby sealing the outlets 7 to the chamber 1, while the o-rings 27 in the recesses 25 ensure that the capillaries 22 are in sealed communication with the receiving chamber 2 via the drain passages 26.

Washing liquid is introduced into the supply chamber 1 through the opening 5, the outlet 28 of the receiving chamber 2 is attached to the vacuum source, and the vacuum is applied. Suction due to the vacuum causes washing liquid to be drawn through the outlet 7 into the cups 23, if used, or into the wells 20, passing through the filter paper discs therein, and hence through the outlet holes 24, capillaries 22 and a drain passage 26, and into receiving chamber 2.

The filter discs can be partly dried using the device, for example by isolating the device from the vacuum source after emptying the supply chamber 1 of washing liquid, to allow the vacuum to build up and then reconnecting the device to the vacuum source so that the rush of air through the filter paper discs causes them to be partly dried. The discs are then removed from the holding plate by removing the cups 23, and radioactive counting can be conducted on the discs while they are still in the cups 23.

In an alternative technique, instead of radio active labelling of the antigen, enzyme labelling is used. The resulting bound enzymes are then used to catalyse a reaction producing a colour change which can then be detected. For such a test, the cups are conveniently placed into receiving members (eg. wells in a conventional titre plate) arranged to seal the holes 24 so that the liquid poured into the cups does not escape.

The washing device of this aspect of the invention is conveniently utilized in the immunoassay methods of the invention.

## Claims

1. A method of carrying out a radio allergosorbent test (RAST test), or a paper radio immunoabsorbent test in which an antibody to IgE is bound to a paper disc (PRIST test), comprising contacting a sample liquid containing an analyte with an absorbent carrier having a binding material immobilised therein or thereon, whereby analyte in the liquid becomes bound to the binding material, allowing a radioactivity labelled indicator to contact the bound analyte, whereby indicator becomes bound to the bound analyte, removing unbound indicator from the carrier, and detecting the remaining bound indicator by detecting the radioactivity thereof, the labelled indicator being contained in a volume of liquid less than ten times the liquid saturation volume of the carrier, characterised in that:
(a) the volume of indicator is not more than 25 µl; and
(b) the concentration of labelled indicator used is such that the amount of indicator present in the volume of liquid is the same as the amount used in a RAST or PRIST test.

2. A method according to claim 1 wherein the volume of indicator is not more than 10 µl.

3. A method according to claim 1 wherein the volume of indicator is not more than 5 µl.

4. A method according to any of claims 1 to 3 wherein the volume of sample liquid is not more than 25 µl.

5. A method according to any of claims 1 to 3, wherein the volume of sample liquid is not more than 10 µl.

6. A method according to any of claims 1 to 3, wherein the volume of the sample liquid is not more than 5 µl.

7. A method according to any of claims 1 to 6, wherein the saturation volume of the carrier is 10 µl or less.

8. A method according to any of claims 1 to 6, wherein the saturation volume of the carrier is 5 µl or less.

9. A method according to any preceding claim, wherein the indicator is contained in a volume of liquid less than five times the saturation volume of the carrier.

10. A method according to any of claims 1 to 8 wherein the indicator is contained in a volume of liquid less than twice the saturation volume of the carrier.

11. A method according to any of claims 1 to 8, wherein the indicator is contained in a volume of liquid substantially equal to the saturation volume of the carrier.

12. A method according to any preceding claim wherein the volume of the sample liquid contacted with the absorbent carrier is less than ten times the saturation volume of the carrier.

13. A method according to any of claims 1 to 11, wherein the volume of the sample liquid contacted with the absorbent carrier is less than five times the saturation volume of the carrier.

14. A method according to any of claims 1 to 11, wherein the volume of the sample liquid contacted with the absorbent carrier is less than twice the saturation volume of the carrier.

15. A method according to any of claims 1 to 11, wherein the volume of the sample liquid contacted with the absorbent carrier is substantially equal to the saturation volume of the carrier.

16. A method according to any preceding claim, characterised in that (a) the radio-labelled indicator has a specific activity of less than 5 µCi/µg; and (b) the radio-labelled indicator has sufficient radioactivity to permit counting of the radioactivity to detect remaining bound indicator in 1 to 2 minutes.

17. A method according to any preceding claim, characterised by the steps of:
applying the sample liquid to the carrier and incubating them together;
washing the sample liquid from the carrier and substantially drying the carrier;
applying to the carrier a liquid containing the indicator and incubating them together;
washing the sample liquid from the carrier; and then detecting the bound indicator.

18. A method according to any preceding claim, characterised by washing and substantially drying the carrier before bringing the carrier and the sample liquid into contact with each other.

19. Use of a washing device in a method according to any of claims 1 to 18, comprising a washing liquid supply chamber (1), a washing liquid receiving chamber (2), and a holding plate (30), therebetween, the holding plate (30) comprising a plurality of wells (20) in the upper side thereof, and each well (20) having an outlet (22) communicating with the underside of the holding plate (30), characterised in that the holding plate (30) is removably mountable between the supply and the receiving chamber (1, 2) and in that (a) a holder (23) is removably located in each well (20), the holder (23) having a drain hole (24) therein corresponding with the outlet, and in that washing liquid supply chamber (1) is arranged to supply liquid to the holders (23), and/or (b) the drain hole (24) in each holder (23) is sized such that liquid in the holder is not lost therethrough under gravity.

20. Use of an immunoassay kit in a method according to any of claims 1 to 18, comprising a plurality of absorbent carrier members having a binding material immobilised therein or thereon, a supply of a solution containing a labelled indicator, and a plurality of different reference samples containing known concentrations of known analytes.

## Patentansprüche

1. Verfahren zur Durchführung eines Radioallergosorbent-Tests (RAST-Test) oder eines Papier-Radioimmunabsorbent-Tests, bei dem ein Antikörper gegen IgE an eine Papierscheibe gebunden wird (PRIST-Test), das darin besteht, daß eine einen Analyt enthaltende Probeflüssigkeit mit einem absorbierenden Träger, in dem oder auf dem ein Bindematerial immobilisiert ist, in Kontakt gebracht wird, wodurch der Analyt in der Flüssigkeit an das Bindematerial gebunden wird, ein radioaktiv markierter Indikator mit dem gebundenen Analyten in Kontakt gebracht wird, wodurch der Indikator an den gebundenen Analyten gebunden wird, der nicht gebundene Indikator von dem Träger entfernt und der verbliebene gebundene Indikator durch Messen seiner Radioaktivität nachgewiesen wird, wobei der markierte Indikator in einem Flüssigkeitsvolumen enthalten ist, das weniger als das Zehnfache des Flüssigkeits-Sättigungsvolumens des Trägers beträgt,
dadurch gekennzeichnet, daß
(a) das Volumen des Indikators nicht mehr als 25 µl ist und
(b) die Konzentration des verwendeten markierten Indikators derart ist, daß die in dem Flüssigkeitsvolumen vorhandene Menge des Indikators die gleiche wie die in einem RAST-Test oder PRIST-Test verwendete Menge ist.

2. Verfahren gemäß Anspruch 1, bei dem das Volumen des Indikators nicht mehr als 10 µl beträgt.

3. Verfahren gemäß Anspruch 1, bei dem das Volumen des Indikators nicht mehr als 5 µl beträgt.

4. Verfahren gemäß einem der Ansprüche 1 bis 3, bei dem das Volumen der Probeflüssigkeit nicht mehr als 25 µl beträgt.

5. Verfahren gemäß einem der Ansprüche 1 bis 3, bei dem das Volumen der Probeflüssigkeit nicht mehr als 10 µl beträgt.

6. Verfahren gemäß einem der Ansprüche 1 bis 3, bei dem das Volumen der Probeflüssigkeit nicht mehr als 5 µl beträgt.

7. Verfahren gemäß einem der Ansprüche 1 bis 6, bei dem das Sättigungsvolumen des Trägers 10 µl oder weniger beträgt.

8. Verfahren gemäß einem der Ansprüche 1 bis 6, bei dem das Sättigungsvolumen des Trägers 5 µl oder weniger beträgt.

9. Verfahren gemäß einem vorhergehenden Anspruch, bei dem der Indikator in einem Flüssigkeitsvolumen enthalten ist, das weniger als das Fünffache des Sättigungsvolumen des Trägers ist.

10. Verfahren gemäß einem der Ansprüche 1 bis 8, bei dem der Indikator in einem Flüssigkeitsvolumen enthalten ist, das weniger als das Zweifache des Sättigungsvolumens des Trägers ist.

11. Verfahren gemäß einem der Ansprüche 1 bis 8, bei dem der Indikator in einem Flüssigkeitsvolumen enthalten ist, das im wesentlichen gleich dem Sättigungsvolumen des Trägers ist.

12. Verfahren nach einem vorhergehenden Anspruch, bei dem das Volumen der Probeflüssigkeit, die in Kontakt mit dem absorbierenden Träger gebracht wird, weniger als das Zehnfache des Sättigungsvolumens des Trägers ist.

13. Verfahren nach einem der Ansprüche 1 bis 11, bei dem das Volumen der Probeflüssigkeit, die in Kontakt mit dem absorbierenden Träger gebracht wird, weniger als das Fünffache des Sättigungsvolumens des Trägers ist.

14. Verfahren nach einem der Ansprüche 1 bis 11, bei dem das Volumen der Probeflüssigkeit, die in Kontakt mit dem absorbierenden Träger gebracht wird, weniger als das Zweifache des Sättigungsvolumens des Trägers ist.

15. Verfahren nach einem der Ansprüche 1 bis 11, bei dem das Volumen der Probeflüssigkeit die mit dem absorbierenden Träger in Kontakt gebracht wird, im wesentlichen gleich dem Sättigungsvolumen des Trägers ist.

16. Verfahren nach einem vorhergehenden Anspruch, dadurch gekennzeichnet, daß (a) der radioaktiv markierte Indikator eine spezifische Aktivität von weniger als 5µCi/µg hat und (b) der radioaktiv markierte Indikator eine zum Auszählen der Radioaktivität ausreichende Radioaktivität hat, die es ermöglicht, den verbliebenen gebundenen Indikator in 1 bis 2 Minuten nachzuweisen.

17. Verfahren nach einem vorhergehenden Anspruch, gekennzeichnet durch folgende Stufen:
Aufbringen der Probeflüssigkeit auf den Träger und gemeinsames Inkubieren dieser,
Abwaschen der Probeflüssigkeit von dem Träger und im wesentlichen Trocknen des Trägers,
Aufbringen einer den Indikator enthaltenden Flüssigkeit auf den Träger und gemeinsames Inkubieren dieser,
Abwaschen der Probeflüssigkeit von dem Träger und schließlich Nachweisen des gebundenen Indikators.

18. Verfahren nach einem vorhergehenden Patentanspruch, dadurch gekennzeichnet, daß der Träger gewaschen und im wesentlichen getrocknet wird, bevor der Träger und die Probeflüssigkeit miteinander in Kontakt gebracht werden.

19. Verwendung einer Waschvorrichtung in einem Verfahren gemäß einem der Ansprüche 1 bis 18, die umfaßt: eine Vorratskammer für die Waschflüssigkeit (1), eine Kammer (2) zur Aufnahme der Waschflüssigkeit und zwischen beiden eine Halteplatte (30), wobei die Halteplatte (30) zahlreiche Vertiefungen (20) in ihrer Oberseite aufweist und jede Vertiefung (20) einen Auslaß (22) hat, der mit der Unterseite der Halteplatte (30) kommuniziert, dadurch gekennzeichnet, daß die Halteplatte (30) abnehmbar zwischen der Vorrats- und der Aufnahmekammer (1, 2) angebracht ist und daß (a) ein Halter (23) abnehmbar in jeder Vertiefung (20) vorgesehen ist, wobei in dem Halter (23) ein Ablauf (24) vorgesehen ist, der mit dem Auslaß in Verbindung steht, und daß die Vorratskammer für die Waschflüssigkeit (1) so angeordnet ist, daß Flüssigkeit zu den Haltern (23) geleitet wird und/oder (b) der Ablauf (24) in jedem Halter (23) so bemessen ist, daß durch diesen kein Verlust von Flüssigkeit in dem Halter unter der Schwerkraft eintritt.

20. Verwendung eines Immunassay-Kits in einem Verfahren gemäß einem der Ansprüche 1 bis 18, welches zahlreiche absorbierende Träger, in denen oder auf denen ein Bindematerial immobilisiert ist, einen Vorrat einer einen markierten Indikator enthaltenden Lösung und zahlreiche verschiedene Referenzproben umfaßt, die bekannte Konzentrationen von bekannten Analyten enthalten.

## Revendications

1. Procédé de mise en oeuvre d'un test allergoabsorbant radioactif (test de RAST), ou d'un test immunoabsorbant radioactif sur papier dans lequel un anticorps de l'IgE est fixé à un disque de papier (test de PRIST), consistant à mettre en contact un échantillon liquide contenant un produit à analyser, avec un porteur absorbant comportant un matériau de fixation immobilisé dans celui-ci ou sur celui-ci, de façon que le produit à analyser contenu dans le liquide est lié au matériau de fixation, à permettre à un indicateur marqué par radioactivité de venir en contact avec le produit à analyser fixé, de façon que l'indicateur soit lié au produit à analyser fixé, à retirer du porteur l'indicateur non fixé et à détecter l'indicateur fixé restant en détectant sa radioactivité, l'indicateur marqué étant contenu dans un volume de liquide inférieur à dix fois le volume de saturation liquide du porteur, procédé caractérisé en ce que :
(a) le volume de l'indicateur ne dépasse pas 25 microlitres ; et
(b) la concentration de l'indicateur marqué utilisé est telle que la quantité d'indicateur présente dans le volume de liquide est la même que la quantité utilisée dans le test de RAST ou le test de PRIST.

2. Procédé selon la revendication 1, caractérisé en ce que le volume de l'indicateur ne dépasse pas 10 microlitres.

3. Procédé selon la revendication 1, caractérisé en ce que le volume d'indicateur ne dépasse pas 5 microlitres.

4. Procédé selon l'une quelconque des revendications 1 à 3, caractérisé en ce que le volume de l'échantillon liquide ne dépasse pas 25 microlitres.

5. Procédé selon l'une quelconque des revendications 1 à 3, caractérisé en ce que le volume de l'échantillon liquide ne dépasse pas 10 microlitres.

6. Procédé selon l'une quelconque des revendications 1 à 3, caractérisé en ce que le volume de l'échantillon liquide ne dépasse pas 5 microlitres.

7. Procédé selon l'une quelconque des revendications 1 à 6, caractérisé en ce que le volume de saturation du porteur est de 10 microlitres ou moins.

8. Procédé selon l'une quelconque des revendications 1 à 6, caractérisé en ce que le volume de saturation du porteur est de 5 microlitres ou moins.

9. Procédé selon l'une quelconque des revendications précédentes, caractérisé en ce que l'indicateur est contenu dans un volume de liquide inférieur à cinq fois le volume de saturation du porteur.

10. Procédé selon l'une quelconque des revendications 1 à 8, caractérisé en ce que l'indicateur est contenu dans un volume de liquide inférieur à deux fois le volume de saturation du porteur.

11. Procédé selon l'une quelconque des revendications 1 à 8, caractérisé en ce que l'indicateur est contenu dans un volume de liquide sensiblement égal au volume de saturation du porteur.

12. Procédé selon l'une quelconque des revendications précédentes, caractérisé en ce que le volume de l'échantillon liquide amené en contact avec le porteur absorbant est inférieur à 10 fois le volume de saturation du porteur.

13. Procédé selon l'une quelconque des revendications 1 à 11, caractérisé en ce que le volume de l'échantillon liquide amené en contact avec le porteur absorbant est inférieur à cinq fois le volume de saturation du porteur.

14. Procédé selon l'une quelconque des revendications 1 à 11, caractérisé en ce que le volume de l'échantillon liquide amené en contact avec le porteur absorbant est inférieur à deux fois le volume de saturation du porteur.

15. Procédé selon l'une quelconque des revendications 1 à 11, caractérisé en ce que le volume de l'échantillon liquide amené en contact avec le porteur absorbant est sensiblement égal au volume de saturation du porteur.

16. Procédé selon l'une quelconque des revendications précédentes, caractérisé en ce que (a) l'indicateur marqué par radioactivité présente une activité spécifique de moins de 5 micro Ci/microgramme ; et (b) l'indicateur marqué par radioactivité présente une radioactivité suffisante pour permettre le comptage de la radioactivité de manière à détecter l'indicateur fixé restant en 1 à 2 minutes.

17. Procédé selon l'une quelconque des revendications précédentes, caractérisé en ce qu'il comprend les différentes étapes consistant à :
- appliquer l'échantillon liquide au porteur et les faire incuber ensemble ;
- laver l'échantillon liquide du porteur et sécher pratiquement complètement le porteur ;
- appliquer au porteur un liquide contenant l'indicateur et les faire incuber ensemble ;
- laver l'échantillon liquide du porteur ; et
- détecter ensuite l'indicateur fixé.

18. Procédé selon l'une quelconque des revendications précédentes, caractérisé en ce qu'il consiste à laver et sécher pratiquement complètement le porteur avant d'amener ce porteur et l'échantillon liquide en contact l'un avec l'autre.

19. Utilisation d'un dispositif de lavage dans un procédé selon l'une quelconque des revendications 1 à 18, comprenant une chambre de fourniture de liquide de lavage (1), une chambre de réception de liquide de lavage (2) et une plaque de maintien (30) entre ces deux chambres, la plaque de maintien (30) comprenant un certain nombre de puits (20) de son côté supérieur et chaque puits (20) comportant un orifice de sortie (22) communiquant avec le côté inférieur de la plaque de maintien (30), utilisation caractérisée en ce que la plaque de maintien (30) peut se monter de manière amovible entre la chambre de fourniture (1) et la chambre de réception (2), et en ce que
(a) un support (23) est placé de manière amovible dans chaque puits (20), ce support (23) comportant un trou de vidange (24) percé dans celui-ci et correspondant à l'orifice de sortie, et la chambre de fourniture de liquide de lavage (1) étant disposée de manière à fournir le liquide de lavage aux supports (23) ; et/ou
(b) le trou de vidange (24) de chaque support (23) est dimensionné de façon que le liquide de lavage contenu dans le support ne se perde pas à travers celui-ci sous l'effet de la pesanteur.

20. Utilisation d'un nécessaire de test immunologique dans un procédé selon l'une quelconque des revendications 1 à 18, utilisation caractérisée en ce qu'elle comprend un certain nombre d'éléments porteurs absorbants comportant un matériau de fixation immobilisé dans ceux-ci ou sur ceux-ci, une alimentation d'une solution contenant un indicateur marqué, et un certain nombre d'échantillons de référence contenant des concentrations connues de produits à analyser connus.
